# EUROPEAN PATENT APPLICATION

(11) **EP 1 517 140 A2**
(43) Date of publication of application: **23.03.2005**
(21) Application number: 03006081.8
(22) Date of filing: 19.03.2003
(51) Int. Cl.: G01N 29/02

(54) **Method and device for diagnostic investigation of biological samples**

(71) Applicant: TF Instruments GmbH, 69120 Heidelberg (DE)
(72) Inventor: Funck, Theodor, 37077 Göttingen (DE)
(74) Representative: Hertz, Oliver, Dr.

(57) **Abstract**

Methods of diagnostic investigation of a sample from a biological organism are described, which comprise the steps of determining at least one physical quantity of said sample,
wherein said at least one physical quantity characterizes an interaction of said sample with sound waves, and correlating said at least one physical quantity with reference data,
which characterize at least one condition of said sample or said organism, for obtaining at least one diagnostic characteristic. Furthermore, diagnostic devices for investigating biological samples with such methods are described.

## Description

### Field of the invention

The invention concerns methods and devices for investigations and diagnostic evaluations of biological samples.

### Technical Background

In human and veterinary medicine, important diagnostic methods are based on qualitative detection or quantitative determination of specific components or marker substances in samples obtained from organisms to be investigated. The samples are taken or prepared from body tissue or body liquids such as blood, serum, liquor, cerebrospinal fluid or the like. Typically, results of chemical-analytical, electrical, magnetic or spectrometric methods are used as basis for diagnostic decisions. As an example, cancer can be detected on the basis of antibody reactions of specific components even during early phases of the disease.

General drawbacks of the conventional diagnostic methods consist of the following features: either their technique is rather simple and therefore their results considerably unspecific and not highly indicative, or they require relative high efforts for detection and quantitative estimation of characteristic components, required for diagnostic correlations.

A typical analytical method for body fluids is their spectrometric investigation, which is often impeded by the turbidity of the sample. As an example, the protein concentration in cerebrospinal fluid can be determined by measuring the optical density (OD) at λ = 280 nm. The extinction value in the sample is increasing with increasing protein contents in the sample. However, the specificity of the extinction value is insufficient for a reliable correlation of this value with pathological situations.

### Summary of the invention

It is the object of the invention to provide new diagnostic methods and devices facilitating the collection of diagnostic characteristics even for complex diseases of human being or animals.

This object is achieved by methods and devices with the features defined in claim 1 resp. claim 15. Advantageous embodiments and applications of the invention are characterized in the dependent claims.

According to a first aspect of the invention, a diagnostic method comprises the steps of determining and evaluating at least one macroscopic physical quantity of a biological sample, wherein this quantity characterizes a mechanical or thermodynamic property (in particular a sound parameter) of said sample and permits the direct derivation of at least one diagnostic information of the sample or a corresponding biological organism. The inventors have found that surprisingly a high-resolution measurement of the at least one physical quantity allows a determination of diagnostic characteristic with high sensitivity. It has been found that changes in the presence and concentration of certain components as caused by diseases or any other particular condition of the organism to be diagnosed sensitively influence the macroscopic mechanical or thermodynamic property of the sample. Due to the unexpected high specificity of the results, diagnostic insight may be directly derived from the measured physical quantity with high precision and reproducibility.

In the context of the present specification, a biological sample or a sample from a biological organism generally indicates a sample, which has been obtained from a living human being, animal or plant. The biological sample preferably comprises a fluid from the body of a human being or an animal, possibly produced by processing or preparing tissue or fluid (e.g. blood, cerebrospinal fluid) of the organism.

Conventional diagnostic methods are mainly directed to the determination of properties on the molecular level, such as the occurrence or the concentration of a marker substance or a component which are characteristic for certain pathological situations. However, the basis of the present invention is the use of certain macroscopic physical properties in which minor but essential components are sensitively projected. A macroscopic physical property comprises a property, which is a characteristic of the whole sample, i. e. a collective property of all atoms or molecules forming the sample.

Preferably, the evaluating of said one or more physical quantities is provided by correlating said quantities with reference data, which characterize at least one condition of said sample or said organism, for obtaining at least one diagnostic information. The reference data used for this correlation, may comprise further measured data (such as e. g. optical density) or empiric data collected from individuals with the diseases to be diagnosed.

The term diagnostic characteristic or diagnostic information indicates any information or chemical/physical data being inherently and directly related to a particular pathologic condition (disease or group of diseases). In this context, conventional diagnostic methods on the basis of measuring physical quantities as e. g. the measurement of blood coagulation do not lead to diagnostic data which are characteristic for a particular disease.

According to preferred embodiments of the invention, the at least one physical quantity to be measured belongs to a group of physical parameters closely related to the mechanical or thermodynamic properties of the sample. Generally, the at least one physical quantity characterizes an interaction of the sample with sound waves. Preferably, the quantities comprise sound velocity, sound absorption, and directly related quantities, such as e. g. at least one resonance frequency of an acoustical resonator, the wavelengths of sound waves, compressibility, mass density, and the refractive index of sound waves. A particular advantage of measuring these quantities is the availability of corresponding high-resolution measurements methods.

Preferably, the at least one physical quantity is measured with a relative precision better than 10⁻³, but a relative precision better than 10⁻⁴ down to 10⁻⁶ is particularly preferred. The relative precision of e. g. 10⁻³ means a measurement of the at least one physical quantity with a systematic and statistical error lower than 0,1 %. The measurement with the relative precision defined above have the particular advantage of an improved specificity and reproducibility of the derived diagnostic correlations.

According to a further preferred embodiment of the invention, at least two physical quantities, preferably a series of physical quantities is measured, while the actual condition of the sample is varied. As an example, the physical quantities are measured under variation of temperature and/or pressure of the sample. Advantageously, this modification may lead to an increased sensitivity and specificity.

According to a further variation of the method according to the invention, so-called relative values are measured like a difference or a quotient of sample values and reference sample values. As an example, the differences of sound velocity in a body liquid to be investigated and e. g. water or a buffer solution are measured as the physical quantity to be evaluated according to the invention. By measuring the relative values, the precision of the measurement can be further improved. As a further modification, also the relative values can be measured at different sample conditions, e. g. at different temperatures and/or pressures of the sample and the reference sample.

Preferably, the at least one measured physical quantity measured in the sample is evaluated by comparing this quantity with at least one reference value measured or otherwise obtained in a reference system. This comparison leads advantageously to a direct diagnostic correlation of physical properties of the sample with well established pathological conditions. As an example, the simple comparison of a measured sound velocity or sound absorption (or a corresponding relative value or related data) with a predetermined threshold value, a pathological situation may be detected and its course predicted. The provision of a comparing step offers advantages with regard to the structure and control of a diagnostic device implementing the method of the invention.

According to a particularly preferred embodiment of the invention, the process of obtaining the at least one physical quantity comprises the qualitative and in some cases quantitative detection of at least one significant component in the sample. Such significant components are biomolecules, e.g. proteins, polysaccharides, lipides, biopolymers, which are specifically characteristic for a certain pathological condition and may be detected by measuring the at least one physical quantity. Advantageously, the invention provides such characteristic information in the form of global physical quantities. Before the invention, such information was available by complex analytical methods only.

The detection of physical properties influenced by biomolecules is particularly preferred. It has been found that the macroscopic physical properties of aqueous systems containing biomolecules are sensitively dependent on the type, the structure and the association properties of the biomolecules. Biomolecules develop interactions with the surrounding water molecules, which sensitively depend on the sample conditions such as temperature or pressure. In other words, a hydration layer is formed around the biomolecules, which is determining the mechanical and thermodynamic properties of the sample fluid. All processes on the molecular level, characterized by modifications of the hydration, such as protonation, deprotonation, dissociation, structure changes, associations and aggregations, are quantitatively detectable by the measurement according to the invention. If a mixture of biomolecules is to be detected in the sample, the effects of the different biomolecules may be separated by a variation of the measurement conditions (e.g. temperature).

Particular advantages arise, if a pathological condition can be directly detected from the at least one measured global physical quantity, compared with the labour-intensive and complex process of specific determination of marker components. This embodiment of the invention can be in particular implemented with neurodegenerative diseases characterized by characteristic biomolecules in a body liquid, like e. g. Alzheimer disease (AD), Creutzfeld Jacob disease (CJD), Multiple Sclerosis (MS), Parkinson disease, Bovine Spongiforme Enzephalopathie (BSE), endogenous depression and the like.

According to a further variation of the invention, a preparation step can be provided when required before the measuring step. The preparation, which comprises e. g. an addition of an additive, a purification or concentration of the sample, and/or a separation of at least one component from the sample enables the measurement of the physical quantity with increased precision and resolution.

According to a second aspect of the invention, a diagnostic device for implementing the diagnostic method of the invention is provided. The diagnostic device comprises a measuring device for measuring the at least one macroscopic physical quantity as well as an evaluating device for evaluating the measured value and obtaining the at least one diagnostic correlation. It is a particular advantage of the invention that the operation of this device may be fully automatic and therefore highly convenient compared with conventional analytic procedures.

The measuring device preferably comprises a resonator system for measuring the sound parameters of the sample. It is preferably equipped with devices enabling variation and control of temperature and/or pressure. Using such a device, the relevant mechanical or thermodynamic properties outlined above can be measured.

Another subject of the invention is a method of using high-resolution measurements (relative precision better than 10⁻³) of sound velocity and related values for detecting diseases.

The invention has the following essential advantages. The invention permits a diagnostic investigation independently of any specific features of the person (e.g. antibody reactions) being investigated. The diagnostic investigation of the invention represents a universal method. Furthermore, the present diagnostic investigation permits a differential diagnosis. Conventional procedures lead to an indication whether a person has a certain disease or not. In contrast, the invention provides an indication of one of various pathologic conditions with one measurement only.

### Brief description of the drawings

The invention will now be described for the purpose of exemplification with reference to the accompanying schematic drawings, which illustrate preferred embodiments and which show in:
- Figure 1:: a schematic illustration of features of diagnostic investigation methods according to preferred embodiments of the invention;
- Figure 2:: a schematic illustration of a diagnostic device according to preferred embodiments of the invention;
- Figure 3:: a correlation diagram illustrating the high selectivity of the diagnostic method according to the invention; and
- Figures 4 to 6:: diagrams of the results of measurements of the ultrasonic velocity during temperature scans in CSF samples of healthy human beings and patients with various diseases.

### Preferred embodiments of the invention

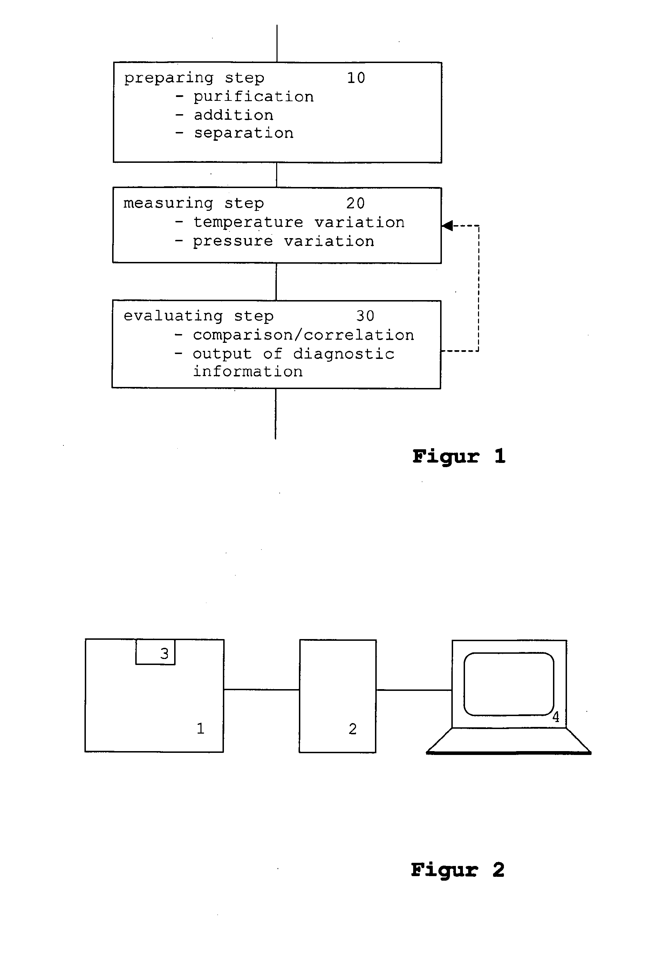

The method and the device according to the invention are basically illustrated in Figures 1 and 2. As described below, the physical properties, which characterize the interaction of the sound wave with the sample, are the preferred physical quantities measured within the scope of the invention.

In a preferred embodiment of the invention, a preparing step 10 is carried out as a first step as shown in Fig. 1. After taking a sample from an organism, the sample may consist of an aqueous fluid containing besides the biomolecular substances, essential for the significant physical properties, cells, cell components or simple substances such as salts, amino acids, small peptides or other organic molecules. The preparing step may comprise e. g. a purification, possibly with a separation of certain components or the addition of substances. The added substances may be intended for e. g. triggering specific reactions with the essential biomolecular substances in the sample. It is emphasized that the invention can be operated without the preparing step 10 depending on the particular physical quantity to be measured.

After the sample preparation, the measuring step 20 is conducted. The measuring step 20 comprises the estimation of the physical parameter, e. g. the sound velocity (ultrasonic velocity) with a procedure being known as such. The sound velocity measurements are carried out e.g. with the RESOSCAN ® system (RESONIC Instruments AG, Germany) or the Ultrasonic PVT system (RESONIC Instruments AG, Germany). The RESOSCAN ® system allows temperature variations during measurement of sound velocity and sound absorption. The Ultrasonic PVT system measures sound velocity and sound absorption during variations of temperature and pressure. As an example, the RESOSCAN system is used for the measurements as proposed by the operational manual of this device in particular with regard to the cleaning of the resonator cell, control of process parameters and careful operation.

After measuring the sound velocity or a series of sound velocity values, the results are evaluated during the evaluating step 30. On the basis of a comparison or correlation evaluation, diagnostic information is obtained as outlined below. The evaluating step 30 may comprise a determination of the relative precision of the measurement. Depending on this determination, the measuring step 20 could be repeated with modified conditions (see dashed arrow).

According to Fig. 2, a diagnostic device of the invention comprises a measuring device 1 and an evaluating device 2. The measuring device 1 is adapted to accommodate the sample 3 and to measure the appropriate physical value. As an example, the measuring device 1 can be a RESOSCAN system (see above). The evaluating device 2 being adapted for evaluating the measured values and for obtaining the at least one diagnostic information preferably is implemented by a computer, which may be integrated in the control of the measuring device 1. Additionally, a display and control device 4 may be provided for operating the whole system. All components shown in Fig. 2 may be integrated into one single device.

### Experimental results

### (1) Sample preparation

The following experimental data illustrate results obtained with the investigation of liquid samples of liquor cerebrospinalis (Central Spinor Fluid, in the following CSF). CSF samples have been collected from human beings with conventional biopsy methods. As an example 2,0 ml CSF is mixed with 2,0 ml PPS buffer solution (pH 7.4). For sample preparation (preparing step 10), albumin and immunglobulins are separated from CSF. The separation is conducted with the Aurum serum protein Minikit (company BioRad, Germany). As a further step, the contents of the remaining protein is measured photometrically (extinction measurement at λ = 280 nm). After the preparation, the prepared sample is stored at a reduced temperature of 4°C. BioRad buffer solution has been prepared as a reference sample.

The samples (180 µl) or reference samples (180 µl) have been introduced into the resonator cell of the RESOSCAN system with a Hamilton syringe providing the sample without bubbles or inhomogeneous regions. The temperature programs provided with the RESOSCAN-system comprise e. g. a temperature range from 10°C to 60°C scanned with 300 mK/min and from 60°C to 10°C with -300 mK/min.

### (2) Correlation measurements

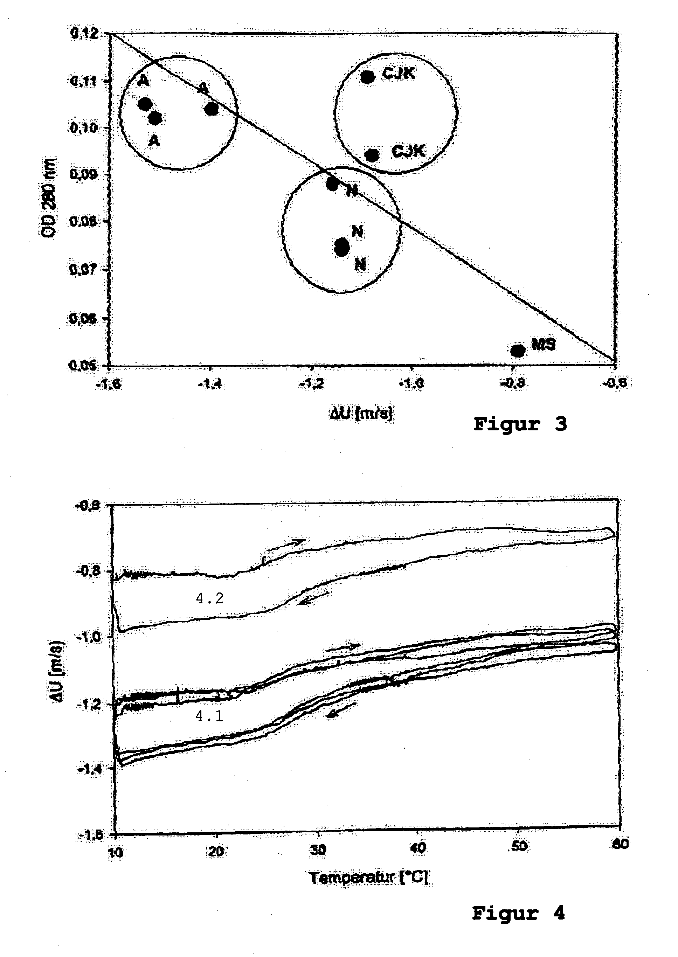

Figure 3 illustrates the correlation of the photometric measurements of the protein contents of a plurality of different samples with corresponding sound velocity quantities. All measurements have been conducted at the same temperature (25°C). Extinction values are used as reference data for the correlation evaluation of the measured velocity quantities. The extinction values (OD) are correlated with relative values of measured sound velocity Δu (Δu represents the difference of sound velocity in a CSF sample and the sound velocity in the reference samples). The CSF samples were obtained from healthy patients (N), and from patients with different diseases, namely, Alzheimer disease (A), Creutzfeld Jacob disease (CJD), Multiple Sclerosis (MS).

The correlation of data of Fig. 3 show that the correlated data are concentrated in different regions of the diagram. The Alzheimer patients show another correlation of optical density and sound velocity compared with the healthy patients or the patients with the other diseases.

Furthermore, Figure 3 shows that correlation of the optical density at 280 nm and the ultrasonic velocity at 25 °C in the samples lead to a strong indication of the various diseases.

### (3) Temperature dependency of sound velocity

In Figures 4 to 7, Δu values are the differences of ultrasonic velocities of sample and buffer solution, resp. The experimental data show that measured relative sound velocities can be used as a direct or indirect measure for a disease producing certain proteins in body liquids, in particular in CSF. In this case, the empirically collected data of patients with respective diseases are used as reference data for the correlation evaluation of the measured velocity quantities.

In Figure 4, the lower group of curves 4.1 comprises the data of healthy persons. The temperature scans from 10°C to 60°C and back to 10°C, in each case shows a difference between the Δu values at increasing and decreasing temperatures. The denaturation of proteins at higher temperatures results in lower Δu values at decreasing temperatures. The upper curve 4.2 represents a sample of a patient with Multiple Sclerosis.

Comparing the data of Fig. 4 with the data of Figs. 3, 5 and 6, resp. shows that measuring the relative ultrasonic velocity even at a single temperature (e. g. at 20°C) could lead to clear indication of Multiple Sclerosis.

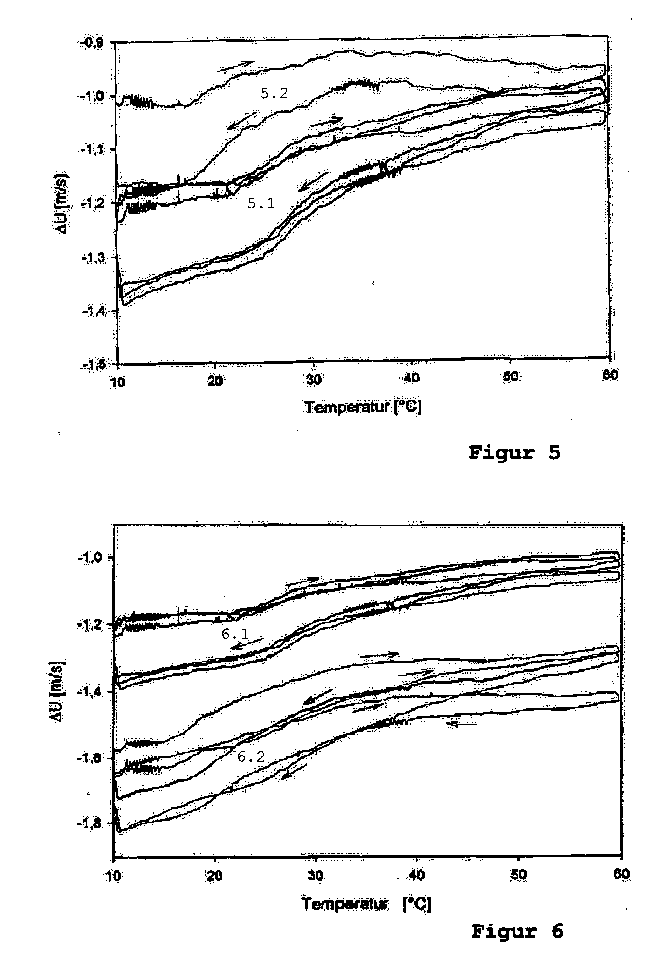

According to Fig. 5, the lower group of curves 5.1 belongs to the healthy persons, while the upper curve 5.2 has been measured with a CSF sample of a patient with endogenous depression. The data show that a selective depression detection is possible on the basis of single measured relative values of sound velocity (e. g. at 25°C) or by evaluating the curve shape. The temperature dependency shows a maximum in both curves 5.2, which can be used as a selective indication of endogenous depression.

According to Fig. 6, the upper group of curves 6.1 shows the data of healthy persons, while the lower group of curves 6.2 are data of samples from patients with Alzheimer disease (compare Fig. 3). The Δu values and the shape of the curves show a characteristic for the Alzheimer disease.

The data show that the content of biomolecules, in particular proteins in the CSF samples influences the ultrasonic velocity. These influences are due to intermolecular interactions between components of the sample. Such intermolecular interactions lead to hydration changes of these components, which cause changes of the mechanical and thermodynamic properties of the sample indicated by changes of the physical quantities characterizing the interaction of the sound wave with the sample. An essential advantage of the invention is given by the surprising observation that the biomolecules being characteristic for pathologic conditions influence the macroscopic behaviour even at low concentrations.

## Claims

1. Method of diagnostic investigation of a sample from a biological organism, comprising the steps of:
- determining at least one physical quantity of said sample,
wherein said at least one physical quantity characterizes an interaction of said sample with sound waves, and
- correlating said at least one physical quantity with reference data, which characterize at least one condition of said sample or said organism, for obtaining at least one diagnostic characteristic.

2. Method of claim 1, wherein said determining step comprises measuring of at least one physical quantity from the group comprising resonance frequency of sound waves, sound wavelength, sound velocity, sound absorption, viscosity, compressibility, mass density, shear wave parameter, acoustic impedance and refractive index of sound waves.

3. Method of claim 1 or 2, wherein said determining step comprises measuring said at least one physical value with a relative precision better than 10⁻³.

4. Method of claim 1 or 2, wherein said determining step comprises measuring at least two physical quantities, said at least two quantities being measured at different temperatures and/or pressures of said sample.

5. Method of claim 1 or 2, wherein said determining step comprises measuring at least one relative quantity of said at least one physical value, said at least one relative quantity being measured as a difference or quotient of a first measured quantity obtained with said sample and a second measured quantity obtained with a reference sample.

6. Method of claim 5, wherein said determining step comprises measuring at least two relative quantities, said at least two relative quantities being measured at different temperatures and/or pressures of said sample and said reference sample, resp..

7. Method of claim 1, wherein said correlating step comprises a step of comparing said one or more physical quantities, a corresponding relative quantity, or a curve shape of said quantities or relative quantities with at least one threshold reference quantity or reference curve shape for obtaining said at least one diagnostic information.

8. Method of claim 1, wherein said step of obtaining of said at least one diagnostic characteristic comprises a detection of at least one biomolecule in said sample.

9. Method of claim 8, wherein said detection comprises a step of determining a presence of at least one protein, lipide or polysaccharide in said sample.

10. Method of claim 1, wherein said step of obtaining of said at least one diagnostic characteristic comprises detecting of a disease of said biological organism.

11. Method of claim 10, wherein said detecting step comprises a step of detecting of at least one disease of the group comprising neurodegenerative diseases producing characteristic biomolecules in a body liquid of said organism.

12. Method of claim 1, further comprising a step of preparing said sample before said determining step, said preparing step comprising one or more from an addition of an additive to said sample, a purification of said sample and a separation of at least one component from said sample.

13. Method of diagnostic investigation of a CSF liquor sample from a human being or an animal, that method comprising the steps of:
- measuring at least one sound velocity value in said prepared sample at at least one temperature and/or pressure, and
- evaluating said at least one value of sound velocity, a corresponding relative value, or a curve shape of said values or relative values and detecting a disease producing predetermined biomolecules in the sample.

14. Method of claim 13, comprising the step of preparing said sample, including separation of albumin and immunglobulines, before said measuring step.

15. Diagnostic device for investigating a sample of a biological organism, said device comprising:
- a measuring device for determining at least one physical quantity of said sample, wherein said at least one physical quantity characterizes an interaction of said sample with sound waves, and
- an evaluating device for evaluating said at least one physical value and for correlating said at least one physical quantity with reference data, which characterize at least one condition of said sample or said organism, for obtaining at least one diagnostic information.

16. Diagnostic device of claim 15, wherein said measuring device comprises a sound resonator cell and a sound frequency detection circuit.

17. Diagnostic device of claim 15, wherein said measuring device comprises a temperature or pressure control device.

18. Diagnostic device of claim 15, wherein said evaluating device comprises a calculating circuit containing a comparison and/or correlation circuit.
